# EUROPEAN PATENT SPECIFICATION

(11) **EP 3 104 940 B1**
(45) Date of publication and mention of the grant of the patent: **06.10.2021**
(21) Application number: 15748932.9
(22) Date of filing: 13.02.2015
(51) Int. Cl.: A61K 31/202, A61K 31/4985, A61P 33/12, A61P 43/00

(54) **ARACHIDONIC ACID AND PRAZIQUANTEL FOR THE PREVENTION AND/OR TREATMENT OF SCHISTOSOMIASIS**
ARACHIDONSÄURE UND PRAZIQUANTEL ZUR VORBEUGUNG UND/ODER BEHANDLUNG VON SCHISTOSOMIASIS
ACIDE ARACHIDONIQUE ET PRAZIQUANTEL POUR PRÉVENIR ET/OU TRAITER UNE SCHISTOSOMIASE

(30) Priority: 14.02.2014 US 201461940041 P
(43) Date of publication of application: 21.12.2016
(73) Proprietor: DSM IP Assets B.V., 6411 TE Heerlen (NL)
(72) Inventor: HADLEY, Kevin, Elkridge, Maryland 21075 (US); EL RIDI, Rashika, Dokki Giza (EG)
(74) Representative: DSM Intellectual Property
(86) International application number: PCT/US2015/015728
(87) International publication number: WO 2015/123480

(56) References cited:
- WO-A1-2013/040206
- WO-A2-2005/055973
- GB-A- 2 460 056
- RASHIKA EL RIDI ET AL: "Efficacy and mechanism of action of arachidonic acid in the treatment of hamsters infected with Schistosoma mansoni or Schistosoma haematobium", INTERNATIONAL JOURNAL OF ANTIMICROBIAL AGENTS, vol. 39, no. 3, March 2012 (2012-03), pages 232-239, XP055381089, AMSTERDAM, NL ISSN: 0924-8579, DOI: 10.1016/j.ijantimicag.2011.08.019
- J. UTZINGER ET AL: "Combination Chemotherapy of Schistosomiasis in Laboratory Studies and Clinical Trials", ANTIMICROBIAL AGENTS AND CHEMOTHERAPY, vol. 47, no. 5, May 2003 (2003-05), pages 1487-1495, XP055381075, ISSN: 0066-4804, DOI: 10.1128/AAC.47.5.1487-1495.2003
- EL RIDI ET AL.: 'Novel Therapeutic and Prevention Approaches for Schistosomiasis: Review.' JOURNAL OF ADVANCED RESEARCH, [Online] vol. 4, no. 5, 2013, ISSN 2090-1232 pages 467 - 478, XP055231273 ISSN: 2090-1232 Retrieved from the Internet: <URL:http://ac.els-cdn.com/S209012321200031 8/1-s2.0-S2090123212000318-main.pdf ? _tid=076a2c82-1f39-11e5-895e-00000aab0f01&a cdnat=1435676730_065810a8c81388f47b273 7c20e79b7a9> [retrieved on 2015-06-30]
- EL RIDI ET AL.: 'In Vitro and In Vivo Activities of Arachidonic Acid against Schistosoma mansoni and Schistosoma haematobium.' ANTIMICROBIAL AGENTS AND CHEMOTHERAPY, [Online] vol. 54, no. 8, 2010, ISSN 0066-4804 pages 3383 - 3389, XP055231276 ISSN: 0066-4804 Retrieved from the Internet: <URL:http://aac.asm.org/content/54/8/3383.f ull.pdf+html> [retrieved on 2015-06-30]
- GARBA. ET AL.: 'Efficacy and safety of two closely spaced doses of praziquantel against Schistosoma haematobium and S. mansoni and re-infection patterns in school-aged children in Niger.' ACTA TROP., [Online] vol. 128, no. 2, 2013, ISSN 0001-706X pages 334 - 344, XP028741601 ISSN: 0001-706X Retrieved from the Internet: <URL:http://www.ncbi.nlm.nih.gov/pubmed/229 40014> [retrieved on 2015-06-30]
- SELIM ET AL.: 'Efficacy and safety of arachidonic acid for treatment of Schistosoma mansoni-infected children in Menoufiya.' AM J TROP MED HYG., [Online] vol. 91, no. 5, November 2014, ISSN 0002-9637 pages 973 - 981, XP055231279 ISSN: 0002-9637 Retrieved from the Internet: <URL:http://www.ncbi.nlm.nih.gov/pubmed/252 46692> [retrieved on 2015-06-30]
- BARAKAT ET AL.: 'Efficacy and Safety of Arachidonic Acid for Treatment of School-Age Children in Schistosoma mansoni High-Endemicity Regions.' AMER. J. TROP. MED. HYG., [Online] vol. 92, no. 4, 2015, ISSN 0002-9637 pages 797 - 804, XP055231280 ISSN: 0002-9637 Retrieved from the Internet: <URL:http://www.ncbi.nlm.nih.gov/pmc/articl es/PMC4385776/pdf/tropmed-92-797.pdf> [retrieved on 2015-06-30]
- None

## Description

### Field of the Invention

Any references in the description to methods of treatment refer to the compounds, pharmaceutical compositions and medicaments of the present invention for use in a method for treatment of the human (or animal) body by therapy (or for diagnosis). Disclosed herein are compositions for the prevention and treatment of schistosomiais comprising a polyunsaturated fatty acid and a therapeutic agent. Further disclosed herein are methods for the prevention and treatment of schistosomiasis, methods to confer resistance to schistosomiasis and methods to prevent re-occurrence of schistosomiasis comprising administration of a polyunsaturated fatty acid and a therapeutic agent.

### Background of the Invention

Schistosomiasis (also known as bilharzia, bilharziosis or snail fever) is a severe parasitic disease caused by several species of fluke of the genus *Schistosoma.* The disease is found predominantly in Asia, Africa and South America and especially in areas with water that are contaminated with snails that carry the parasite. It is the second most common parasitic disease after malaria and affects mainly children. Schistosomiasis is caused by the platyhelminth worms of the genus *Schistosoma,* trematodes that live in the bloodstream of humans and animals. Three species (*Schistosoma mansoni, Schistosoma haematobium* and *Schistosoma japonicum)* account for the majority of the infections. Schistosomes migrate against the direction of blood flow to their permanent abode with S. *masoni* and *S*. *japonicum* to the inferior mesoteric and S. *haematobium* to the peri-vesical venous plexus. Schistosomes lay eggs near the conduit for egg passage to the external environment to complete the life cycle. Accordingly, massive number of eggs exit daily from the blood capillaries to the lumen of the gut or urinary bladder whereby they may be detected by analysis of stool and urine samples.

Arachidonic acid (ARA), all-*cis* 5,8,11,14-eicosatetreanoic acid, an omega-6 fatty acid: 20:4(ω-6), is present in the phospholipids of membranes of the body's cells and has been hypothesized to act to kill juvenile and adult male and female *S*. *masoni* and *S*. *haematobium* worms, see for example EI Ridi et al.: "In vitro and in vivo activities of arachidonic acid against Schistosoma mansoni and Schistosoma haematobium.", Antimicrob. Agents Chemother., vol. 54, no. 8, 2010, pages 3383-3389, EI Ridi et al.: "Efficacy and mechanism of action of arachidonic acid in the treatment of hamsters infected with Schistosoma mansoni or Schistosoma haematobium", Int. J. Antimicrob., vol. 39, no. 3, March 2012 (2012-03), pages 232-239, and GB 2460056A.

Praziquantel (PZQ, for a review see EI Ridi et al.: "Novel therapeutic and prevention approaches for schistosomiasis: Review", J. Adv. Res., vol. 4, no. 5, 2013, pages 467-478) is currently the only drug for treatment of Schistosomiasis, particularly *S*. *masoni.* However, the rate of failure of cure in children following treatment was consistently associated with high intensity of infection, requiring repeated treatments and accelerating the impending threat of schistosomes' resistance to the drug. Further, PZQ was not shown to confer resistance to schistosomes. As a result, there is a need for a composition, alone or in combination with PZQ, for the treatment and/or prevention of Schistosomiasis and to confer resistance to and/or prevent re-occurrence of schistosomiasis.

### Summary of the Invention

The present invention relates to a composition for the treatment and/or prevention of schistosomiasis, comprising at least one polyunsaturated fatty acid (PUFA) and at least one therapeutic compound, wherein the PUFA is arachidonic acid and wherein the therapeutic compound is 2-(Cyclohexylcarbonyl)-1,2,3,6,7,11b-hexahydro-4H-pyrazino[2,1-a]isoquinolin-4-one (praziquantel), wherein the PUFA is in the form of a free fatty acid, salt, fatty acid ester (e.g. methyl or ethyl ester), monoacylglycerol (MAG), diacylglycerol (DAG), triacylglycerol (TAG), and/or phospholipid (PL). The present invention also relates to this composition for use for the treatment and/or prevention of schistosomiasis, to confer resistance to schistosomiasis, and/or to prevent re-occurrence of schistosomiasis.

Preferably, the composition comprises a PUFA that is an omega-6 fatty acid that is all-*cis* 5,8,11,14-eicosatetreanoic acid, also known as arachidonic acid (ARA).

Additionally, the methods disclosed herein comprise administration of a polyunsaturated fatty acid (PUFA) and a therapeutic agent. In one embodiment, the PUFA is administered in one dosage and the therapeutic agent is administered in a second dosage. In another embodiment, the PUFA and the therapeutic agent are administered in the same dosage.

### Detailed Description of the Invention

Disclosed herein are compositions for the prevention and/or treatment of schistosomiais comprising a polyunsaturated fatty acid and a therapeutic agent. Further described herein are methods for the prevention and/or treatment of schistosomiasis, methods to confer resistance to schistosomiasis and methods to prevent re-occurrence of schistosomiasis comprising administration of a polyunsaturated fatty acid and a therapeutic agent in a therapeutically effective amount.

The features and advantages of the invention may be more readily understood by those of ordinary skill in the art upon reading the following detailed description. It is to be appreciated that certain features of the invention that are, for clarity reasons, described above and below in the context of separate embodiments, may also be combined so as to sub-combinations thereof.

Embodiments identified herein as exemplary are intended to be illustrative and not limiting.

The term "about" is intended to capture variations above and below the stated number that may achieve substantially the same results as the stated number.

Fatty acids are classified based on the length and saturation characteristics of the carbon chain. Fatty acids present in a microbial oil can have from 4 to 28 carbon atoms and are termed short chain, medium chain, or long chain fatty acids based on the number of carbons present in the chain. Fatty acids are termed saturated fatty acids when no double bonds are present between the carbon atoms, and are termed unsaturated fatty acids when double bonds are present. Unsaturated long chain fatty acids are monounsaturated when only one double bond is present and are polyunsaturated when more than one double bond is present.

Polyunsaturated fatty acids (PUFAs) are classified based on the position of the first double bond from the methyl end of the fatty acid; omega-3 (n-3) fatty acids contain a first double bond at the third carbon, while omega-6 (n-6) fatty acids contain a first double bond at the sixth carbon. For example, docosahexaenoic acid (DHA) is an omega-3 long chain polyunsaturated fatty acid (LC-PUFA) with a chain length of 22 carbons and 6 double bonds, often designated as "22:6n-3." In one embodiment, the PUFA is selected from an omega-3 fatty acid, an omega-6 fatty acid, and mixtures thereof. In another embodiment, the PUFA is selected from long-chain polyunsaturated fatty acids (LC-PUFAs). In a still further embodiment, the PUFA is selected from docosahexaenoic acid (DHA), eicosapentaenoic acid (EPA), docosapentaenoic acid (DPA), arachidonic acid (ARA), gamma-linolenic acid (GLA), dihomo-gamma-linolenic acid (DGLA), stearidonic acid (SDA), and mixtures thereof. In another embodiment, the PUFA is selected from DHA, ARA, and mixtures thereof. In a further embodiment, the PUFA is DHA. In yet a further embodiment, the PUFA is ARA.

LC-PUFAs are fatty acids that contain at least 3 double bonds and have a chain length of 18 or more carbons or 20 or more carbons. LC-PUFAs of the omega-6 series include, but are not limited to, di-homo-gammalinoleic acid (C20:3n-6), arachidonic acid (C20:4n-6) ("ARA"), docosatetraenoic acid or adrenic acid (C22:4n-6), and docosapentaenoic acid (C22:5n-6) ("DPA n-6"). The LC-PUFAs of the omega-3 series include, but are not limited to, eicosatrienoic acid (C20:3n-3), eicosatetraenoic acid (C20:4n-3), eicosapentaenoic acid (C20:5n-3) ("EPA"), docosapentaenoic acid (C22:5n-3), and docosahexaenoic acid (C22:6n-3). The LC-PUFAs also include fatty acids with greater than 22 carbons and 4 or more double bonds including, but not limited to, C24:6(n-3) and C28:8(n-3).

The PUFAs can be in the form of a free fatty acid, salt, fatty acid ester (e.g. methyl or ethyl ester), monoacylglycerol (MAG), diacylglycerol (DAG), triacylglycerol (TAG), and/or phospholipid (PL).

Any source of PUFA can be used in the compositions and methods of the invention, including, for example, animal, plant and microbial sources. Preferred polyunsaturated fatty acid (PUFA) sources can be any source of PUFA that are suitable for use in the present invention.

Examples of animal sources include aquatic animals (e.g., fish, marine mammals, crustaceans, rotifers, etc.). Examples of plant sources include microalgae, flaxseeds, rapeseeds, corn, evening primrose and borage. Examples of microorganisms include microalgae, protists, bacteria and fungi (including yeast). The use of a microorganism source, such as microalgae, can provide organoleptic advantages.

In accordance with the present invention, the polyunsaturated fatty acids that are used in the compositions described herein are in a variety of forms, for example, such forms include, but are not limited to: a highly purified algal oil comprising a PUFA, a plant oil comprising the PUFA, triglyceride oil comprising the PUFA, phospholipid comprising the PUFA, a combination of protein and phospholipids comprising the PUFA, dried marine microalgae comprising the PUFA, sphingolipids comprising the PUFA, esters of the PUFA, free fatty acid, a conjugate of the PUFA with another bioactive molecule, and conjugates thereof.

In one embodiment, the composition comprises a polyunsaturated fatty acid (PUFA) and a therapeutic agent. Preferably, the composition comprises a PUFA that is an omega-6 fatty acid. More preferably, the composition comprises a PUFA that is an omega-6 fatty acid that is all-*cis* 5,8,11,14-eicosatetreanoic acid, also known as arachidonic acid (ARA). In one embodiment, the composition comprises a therapeutic agent that is a trematocide. Preferably, the composition comprises a therapeutic agent that is a trematocide that is 2-(Cyclohexylcarbonyl)-1,2,3,6,7,11b-hexahydro-4*H*-pyrazino[2,1-*a*]isoquinolin-4-one, also known as Praziquantel (PZQ), or a derivative thereof.

In some embodiments, the PUFA is administered in one dosage and the therapeutic compound is administered in a second dosage.

In some embodiments, the PUFA is administered in an amount of about 5 mg/kg body weight/day, about 7 mg/kg body weight/day, about 10 mg/kg body weight/day, about 12 mg/kg body weight/day, about 15 mg/kg body weight/day, or from about 5 to about 15 mg/kg body weight/day, from about 7 to about 12 mg/kg body weight/day, from about 9 to about 11 mg/kg body weight/day.

In one embodiment, the PUFA is administered in an amount of about 10 mg/kg body weight/day.

In some embodiments, the PUFA is administered in an amount of from about 10 mg/kg body weight/day to about 600 mg/kg body weight/day, from about 20 mg/kg body weight/day to about 550 mg/kg body weight/day, from about 40 mg/kg body weight/day to about 500 mg/kg body weight/day, from about 50 mg/kg body weight/day to about 450 mg/kg body weight/day, from about 60 mg/kg body weight/day to about 400 mg/kg body weight/day, from about 70 mg/kg body weight/day to about 350 mg/kg body weight/day, from about 80 mg/kg body weight/day to about 300 mg/kg body weight/day, from about 90 mg/kg body weight/day to about 250 mg/kg body weight/day, from about 100 mg/kg body weight/day to about 200 mg/kg body weight/day, from about 110 mg/kg body weight/day to about 150 mg/kg body weight/day, about 10 mg/kg body weight/day about 20 mg/kg body weight/day, about 30 mg/kg body weight/day, about 40 mg/kg body weight/day, about 50 mg/kg body weight/day, about 60 mg/kg body weight/day, about 70 mg/kg body weight/day, about 80 mg/kg body weight/day, about 90 mg/kg body weight/day, about 100 mg/kg body weight/day, about 110 mg/kg body weight/day, about 120 mg/kg body weight/day, about 130 mg/kg body weight/day, about 140 mg/kg body weight/day, about 150 mg/kg body weight/day, about 160 mg/kg body weight/day, about 170 mg/kg body weight/day, about 180 mg/kg body weight/day, about 190 mg/kg body weight/day, about 200 mg/kg body weight/day, about 250 mg/kg body weight/day, about 300 mg/kg body weight/day, about 350 mg/kg body weight/day, about 400 mg/kg body weight/day, about 450 mg/kg body weight/day, about 500 mg/kg body weight/day, about 550 mg/kg body weight/day, about 600 mg/kg body weight/day.

In one embodiment, the PUFA is administered in an amount of about 150 mg/kg body weight/day.

In some embodiments, the PUFA is administered in a single dose or multiple doses.

In some embodiments, the PUFA is administered over about 30 days, over about 20 days, over about 15 days, over about 10 days, over about 5 days, or over about 1 to 30 days, over about 1 to 20 days, over about 1 to 15 days, over about 1 to 10 days, over about 1 to 5 days.

In some embodiments, the therapeutic agent is administered in an amount of about 20 mg/kg body weight/day, in an amount of about 25 mg/kg body weight/day, in an amount of about 30 mg/kg body weight/day, in an amount of about 35 mg/kg body weight/day, in an amount of about 40 mg/kg body weight/day, in an amount of about 45 mg/kg body weight/day, in an amount of from about 50 mg/kg body weight/day, in an amount of about 55 mg/kg body weight/day, in an amount of about 60 mg/kg body weight/day, in an amount of from about 20 to about 60 mg/kg body weight/day, in an amount of from about 25 to about 55 mg/kg body weight/day, in an amount of from about 30 to about 50 mg/kg body weight/day, in an amount of from about 35 to about 45 mg/kg body weight/day.

In one embodiment, the therapeutic agent is administered in an amount of about 40 mg/kg body weight/day.

In some embodiments, the Schistosomiasis is *Schistosoma mansoni, Schistosoma haematobium* and *Schistosoma japonicum.* In some embodiments, the Schistosomiasis is *Schistosoma mansoni.*

Additionally, the methods described herein comprise administration of a polyunsaturated fatty acid (PUFA) and a therapeutic agent for the treatment or prevention of schistosomiasis. In another embodiment, the methods disclosed herein comprise administration of a polyunsaturated fatty acid and a therapeutic agent to confer resistance to schistosomiasis. In another embodiment, the methods disclosed herein comprise administration of a polyunsaturated fatty acid and a therapeutic agent to prevent re-occurrence of schistosomiasis.

In some embodiments, the PUFA is an omega-6 fatty acid. More preferably, the PUFA is an omega-6 fatty acid that is all-*cis* 5,8,11,14-eicosatetreanoic acid, also known as arachidonic acid (ARA).

In some embodiments, the therapeutic agent is a trematocide. Preferably, the therapeutic agent that is a trematocide is 2-(Cyclohexylcarbonyl)-1,2,3,6,7,11b-hexahydro-4*H-*pyrazino[2,1-a]isoquinolin-4-one, also known as Praziquantel (PZQ), or a derivative thereof.

In some embodiments, the PUFA is administered in one dosage and the therapeutic compound is administered in a second dosage.

In some embodiments, the PUFA is administered in an amount of from about 5 mg/kg body weight/day, about 7 mg/kg body weight/day, about 10 mg/kg body weight/day, about 12 mg/kg body weight/day, about 15 mg/kg body weight/day or from about 5 to about 15 mg/kg body weight/day, from about 7 to about 12 mg/kg body weight/day, from about 9 to about 11 mg/kg body weight/day.

In one embodiment, the PUFA is administered in an amount of about 10 mg/kg body weight/day.

In some embodiments, the PUFA is administered in an amount of from about 10 mg/kg body weight/day to about 600 mg/kg body weight/day, from about 20 mg/kg body weight/day to about 550 mg/kg body weight/day, from about 40 mg/kg body weight/day to about 500 mg/kg body weight/day, from about 50 mg/kg body weight/day to about 450 mg/kg body weight/day, from about 60 mg/kg body weight/day to about 400 mg/kg body weight/day, from about 70 mg/kg body weight/day to about 350 mg/kg body weight/day, from about 80 mg/kg body weight/day to about 300 mg/kg body weight/day, from about 90 mg/kg body weight/day to about 250 mg/kg body weight/day, from about 100 mg/kg body weight/day to about 200 mg/kg body weight/day, from about 110 mg/kg body weight/day to about 150 mg/kg body weight/day, about 10 mg/kg body weight/day about 20 mg/kg body weight/day, about 30 mg/kg body weight/day, about 40 mg/kg body weight/day, about 50 mg/kg body weight/day, about 60 mg/kg body weight/day, about 70 mg/kg body weight/day, about 80 mg/kg body weight/day, about 90 mg/kg body weight/day, about 100 mg/kg body weight/day, about 110 mg/kg body weight/day, about 120 mg/kg body weight/day, about 130 mg/kg body weight/day, about 140 mg/kg body weight/day, about 150 mg/kg body weight/day, about 160 mg/kg body weight/day, about 170 mg/kg body weight/day, about 180 mg/kg body weight/day, about 190 mg/kg body weight/day, about 200 mg/kg body weight/day, about 250 mg/kg body weight/day, about 300 mg/kg body weight/day, about 350 mg/kg body weight/day, about 400 mg/kg body weight/day, about 450 mg/kg body weight/day, about 500 mg/kg body weight/day, about 550 mg/kg body weight/day, about 600 mg/kg body weight/day.

In one embodiment, the PUFA is administered in an amount of about 150 mg/kg body weight/day.

In some embodiments, the PUFA is administered in a single dose or multiple doses.

In some embodiments, the PUFA is administered over about 30 days, over about 20 days, over about 15 days, over about 10 days, over about 5 days, over about 1 to 30 days, over about 1 to 20 days, over about 1 to 15 days, over about 1 to 10 days, over about 1 to 5 days.

In some embodiments, the therapeutic agent is administered in an amount of about 20 mg/kg body weight/day, in an amount of about 25 mg/kg body weight/day, in an amount of about 30 mg/kg body weight/day, in an amount of about 35 mg/kg body weight/day, in an amount of about 40 mg/kg body weight/day, in an amount of about 45 mg/kg body weight/day, in an amount of about 50 mg/kg body weight/day, in an amount of about 55 mg/kg body weight/day, in an amount of about 60 mg/kg body weight/day, in an amount of from about 20 to about 60 mg/kg body weight/day, in an amount of from about 25 to about 55 mg/kg body weight/day, in an amount of from about 30 to about 50 mg/kg body weight/day, in an amount of from about 35 to about 45 mg/kg body weight/day.

In one embodiment, the therapeutic agent is administered in an amount of about 40 mg/kg body weight/day.

In some embodiments, the Schistosomiasis is *Schistosoma mansoni, Schistosoma haematobium* and *Schistosoma japonicum.* In some embodiments, the Schistosomiasis is *Schistosoma mansoni.*

### EXAMPLES

### Example 1

### Subjects

Approximately 2000 school children in villages of three districts in the Menoufiya governorate located about 100 km north of Cairo, Egypt were screened. Two microscopic slides of stool samples were examined for each child on 3 consecutive days by the Kato-Katz method and egg counts per gram (epg) were recorded. 66 children, 44 males and 22 females, 6-15 years old (mean ± SD = 11.7 ± 1.6), with weight range of 25-52 kg (mean ± SD = 37.8 ± 8.6), and epg of 24-384 were selected as test subjects. 20 sex, age, and social conditions-matched, parasite-free children were used as controls.

### Treatment

The 66 children were divided randomly into three groups whereby the first group of 20 children were given a single oral dose of Praziquantel (PZQ) (40 mg/kg body weight) on the first day of treatment and a placebo was given for the next three weeks (5 doses/week) ("PZQ-Placebo Treatment"). The PZQ was Distocide (Epico, El-Asher-Men-Ramadan City, Egypt). The placebo was 1g VegCap, containing corn-soy (DSM Nutritional Products, Columbia, Maryland). The second group of 23 children received ARA (10 mg/kg body weight/day) for 15 days over three weeks (5 days/ week) ("ARA Treatment") The ARA was 1g VegCap (DSM) containing approximately 395 mg ARA/capsule. The third group of 23 children were given Praziquantel (40 mg/kg body weight) in the first day of treatment and then received 15 doses of ARA (10 mg/kg/day; 5 doses/week) ("PZQ + ARA Treatment").

### Study Design

Approximately 10 ml blood were obtained from each child 2-3 days before the start of the treatment and 3 days after the end of the ARA (or ARA + PZQ) treatment, corresponding to 18 days after PZQ-only treatment. Stool samples were obtained from each child on 3 consecutive days 1 week after the end of the ARA treatment (corresponding to 4 weeks after the PZQ-only treatment) and 6 weeks after the end of the ARA treatment (corresponding to 9 weeks after the PZQ-only treatment). Epg was assessed on each stool sample.

### Biochemical and Hematological Parameters

Samples for biochemical analysis were put in a plain tube and allowed to clot for at least 60 minutes, centrifuged, and the serum was collected. Two ml blood samples were dispensed into a tube with EDTA and analyzed by automated blood hematology analyzer (XT 1800i, Sysmex Corporation).

### Levels of Plasma Interleukin-10 (IL-10) and Interferon-Gamma (IFN-γ)

Plasma was retrieved from heparinized blood following centrifugation at 400 g for 20 minutes and stored at -76°C until assayed by capture enzyme-linked immunosorbent assay (ELISA) for levels of IL-10 and IFN-γ (ELISA MAX™ Set, BioLegend, San Diego, California) according to the manufacturer's protocol.

### Whole Blood Cytokine Response to Parasite Antigens

Heparinized whole blood cells were diluted 1:4 in Roswell Park Memorial Institute (RPMI)-1640 medium supplemented with 200 U/ml penicillin, 200 µg/mL streptomycin, 50 ng/mL amphotericin, and 20 µg/mL polymixin B (Sigma) as an inhibitor of any residual lipopolysaccharide contamination of antigens. 200 µl diluted blood was incubated in duplicate wells of sterile roundbottomed well microtiter plates with 50 µl medium containing 0 or 40 µg/mL recombinant S. *mansoni* glyceraldehyde 3-phosphate dehydrogenase (rSG3PDH). Whole blood cultures were incubated for 72 hours at 37°C/3% CO₂, and then centrifuged at 400 g for 10 minutes. The cell-free supernatants were transferred into wells of sterile plate, and stored at -76°C until assayed by capture ELISA for levels of released IL-4, IL-17 and IFN-γ (ELISA MAX™ Set, BioLegend, San Diego, California) according to the manufacturer's protocol.

### Results

Subjects having initial baseline epg counts < 100 were classified as having low infection. Subjects having initial baseline epg counts > 100 but < 400 were classified as having moderate infection. Subjects having initial baseline epg counts > 400 were classified as having high infection.

All treatment groups had a % cure rate in the moderate infection group less than in the low infection group. The % cure for the PZQ-Placebo moderate and high infection group was 67% as compared to 71% for the low infection group. The % cure for the ARA moderate and high infection group was 50% as compared to 77% for the low infection group. The % cure rate for the PZQ-ARA moderate and high infection group was 71% as compared to 94% for the low infection group. The PZQ-ARA treatment group resulted in a higher % cure rate for the low infection groups versus the PZQ-Placebo treatment group or the ARA treatment group (94%, 71% and 77%, respectively). The % cure rate for the PZQ-ARA treatment group resulted in a higher % cure rate for the moderate and high infection groups versus the PZQ-Placebo treatment group or the ARA treatment group (71%, 67% and 50%, respectively).

### Efficacy of PZQ-Placebo Treatment

**Table 1: PZQ-Placebo Treatment**

| **Subjects with initial baseline epg < 100** | | | |
|---|---|---|---|
| **Subject Number** | **Baseline epg** | **epg After Week 4** | **epg After Week 9** |
| 1 | 96 | 0 | 0 |
| 4 | 24 | 0 | 0 |
| 7 | 24 | 5 | 0 |
| 10 | 48 | 5 | 20 |
| 13 | 48 | ---- | 10 |
| 16 | 24 | 8 | 4 |
| 19 | 48 | 0 | 0 |
| 34 | 72 | 0 | 0 |
| 37 | 24 | 0 | 0 |
| 40 | 48 | 0 | 6 |
| 46 | 48 | 0 | 0 |
| 54 | 96 | 0 | 0 |
| 66 | 96 | 0 | 0 |
| 58 | 48 | ---- | 0 |

As shown in Table 1, the PZQ-Placebo Treatment resulted in a 71% cure rate for children having low infection (10/14 children exhibited 0 epg after 9 weeks).

**Table 2: PZQ- Placebo Treatment**

| **Subjects with initial baseline epg > 100** | | | |
|---|---|---|---|
| **Subject Number** | **Baseline epg** | **epg at Week 4** | **epg at Week 9** |
| 22 | 120 | 0 | 10 |
| 25 | 192 | 16 | 0 |
| 28 | 120 | ---- | 30 |
| 31 | 168 | 0 | 0 |
| 43 | 3312 | ---- | 0 |
| 51 | 192 | 0 | 0 |

As shown in Table 2, the PZQ-Placebo Treatment resulted in a 67% cure rate for children having moderate and high infection (4/6 children exhibited 0 epg after 9 weeks).

### Efficacy of ARA Treatment

**Table 3: ARA Treatment**

| **Subjects with initial baseline epg < 100** | | | |
|---|---|---|---|
| **Subject Number** | **Baseline epg** | **epg After Week 4** | **epg After Week 9** |
| 5 | 96 | 0 | 42 |
| 8 | 48 | 19 | 0 |
| 11 | 24 | 24 | 0 |
| 14 | 24 | 0 | 0 |
| 17 | 24 | 12 | 0 |
| 32 | 72 | ---- | 0 |
| 35 | 96 | 0 | 0 |
| 41 | 96 | 144 | 376 |
| 47 | 96 | 24 | 20 |
| 49 | 48 | 20 | 0 |
| 68 | 48 | 0 | 0 |
| 59 | 96 | 52 | 0 |
| 57 | 96 | 48 | 0 |

As shown in Table 3, the ARA Treatment resulted in a 77% cure rate for children having low infection (10/13 children exhibited 0 epg after week 9).

**Table 4: ARA Treatment**

| **Subjects with initial baseline epg > 100** | | | |
|---|---|---|---|
| **Subject Number** | **Baseline epg** | **epg After Week 4** | **epg After Week 9** |
| 2 | 144 | 96 | 48 |
| 20 | 192 | 48 | 28 |
| 23 | 192 | 60 | 20 |
| 26 | 168 | 96 | 152 |
| 29 | 384 | 240 | 0 |
| 38 | 312 | 96 | 0 |
| 44 | 912 | 749 | 0 |
| 52 | 286 | 216 | 108 |
| 55 | 192 | 84 | 0 |
| 62 | 120 | 0 | 0 |

As shown in Table 4, the ARA Treatment resulted in a 50% cure rate for children having moderate and high infection (5/10).

### Efficacy of PZQ-ARA Treatment

**Table 5: PZQ-ARA Treatment**

| **Subjects with initial baseline epg < 100** | | | |
|---|---|---|---|
| **Subject Number** | **Baseline epg** | **epg After Week 4** | **epg After Week 9** |
| 3 | 48 | 0 | 0 |
| 6 | 96 | 0 | 0 |
| 9 | 72 | 0 | 0 |
| 12 | 24 | 0 | 0 |
| 21 | 72 | 0 | 38 |
| 33 | 96 | 0 | 0 |
| 36 | 24 | 0 | 0 |
| 39 | 96 | 12 | 0 |
| 48 | 96 | 0 | 0 |
| 50 | 72 | 24 | 0 |
| 53 | 96 | 0 | 0 |
| 67 | 72 | 0 | 0 |
| 56 | 96 | 0 | 0 |
| 60 | 48 | 0 | 0 |
| 63 | 96 | 0 | 0 |
| 65 | 72 | 0 | 0 |

As shown in Table 5, the PZQ-ARA Treatment resulted in a 94% cure rate for children having low infection (15/16 children exhibited 0 epg after week 9).

**Table 6: PZQ-ARA Treatment**

| **Subjects with initial baseline epg >100** | | | |
|---|---|---|---|
| **Subject Number** | **Baseline epg** | **epg After Week 4** | **epg After Week 9** |
| 15 | 384 | 248 | 0 |
| 18 | 192 | 88 | 0 |
| 24 | 192 | 0 | 0 |
| 27 | 1800 | 102 | 40 |
| 30 | 672 | 0 | 0 |
| 42 | 288 | 0 | 10 |
| 45 | 120 | 29 | 0 |

As shown in Table 6, the PZQ-ARA Treatment resulted in a 71% cure rate for children having moderate and high infection (5/7 children exhibited 0 epg after week 9).

### Example 2

### Subjects

School children from EI Kafr Sheikh were screened similar to those school children in Example 1 above.

### Treatment

The children were divided randomly into three groups whereby the first group of children were given a single oral dose of Praziquantel (PZQ) (40 mg/kg body weight) on the first day of treatment and a placebo was given for the next three weeks (5 doses/week) ("PZQ-Placebo Treatment"). The PZQ was Distocide (Epico, EI-Asher-Men-Ramadan City, Egypt). The placebo was 1g VegCap, containing corn-soy (DSM Nutritional Products, Columbia, Maryland). The second group of children received ARA (10 mg/kg body weight/day) for 15 days over three weeks (5 days/ week) ("ARA Treatment"). The ARA was 1g VegCap (DSM) containing approximately 395 mg ARA/capsule. The third group of children were given Praziquantel (40 mg/kg body weight) in the first day of treatment and then received 15 doses of ARA (10 mg/kg/day; 5 doses/week) ("PZQ-ARA Treatment").

### Study Design

Approximately 10 ml blood were obtained from each child 2-3 days before the start of the treatment and 3 days after the end of the ARA (or ARA + PZQ) treatment, corresponding to 18 days after PZQ-only treatment. Stool samples were obtained from each child on 3 consecutive days 1 week after the end of the ARA treatment (corresponding to 4 weeks after the PZQ-only treatment) and 6 weeks after the end of the ARA treatment (corresponding to 9 weeks after the PZQ-only treatment). Epg was assessed on each stool sample.

### Biochemical and Hematological Parameters

Samples for biochemical analysis were put in a plain tube and allowed to clot for at least 60 minutes, centrifuged, and the serum was collected. Two ml blood samples were dispensed into a tube with EDTA and analyzed by automated blood hematology analyzer (XT 1800i, Sysmex Corporation).

### Levels of Plasma Interleukin-10 (IL-10) and Interferon-Gamma (IFN-γ)

Plasma was retrieved from heparinized blood following centrifugation at 400 g for 20 minutes and stored at -76°C until assayed by capture enzyme-linked immunosorbent assay (ELISA) for levels of IL-10 and IFN-γ (ELISA MAX™ Set, BioLegend, San Diego, California) according to the manufacturer's protocol.

### Whole Blood Cytokine Response to Parasite Antigens

Heparinized whole blood cells were diluted 1:4 in Roswell Park Memorial Institute (RPMI)-1640 medium supplemented with 200 U/ml penicillin, 200 µg/mL streptomycin, 50 ng/mL amphotericin, and 20 µg/mL polymixin B (Sigma) as an inhibitor of any residual lipopolysaccharide contamination of antigens. 200 µl diluted blood was incubated in duplicate wells of sterile roundbottomed well microtiter plates with 50 µl medium containing 0 or 40 µg/mL recombinant S. *mansoni* glyceraldehyde 3-phosphate dehydrogenase (rSG3PDH). Whole blood cultures were incubated for 72 hours at 37°C/3% CO₂, and then centrifuged at 400 g for 10 minutes. The cell-free supernatants were transferred into wells of sterile plate, and stored at -76°C until assayed by capture ELISA for levels of released IL-4, IL-17 and IFN-γ (ELISA MAX™ Set, BioLegend, San Diego, California) according to the manufacturer's protocol.

### Results

Subjects having initial baseline epg counts < 100 were classified as having low infection. Subjects having initial baseline epg counts > 100 but < 400 were classified as having moderate infection. Subjects having initial baseline epg counts > 400 were classified as having high infection.

All treatment groups had a % cure rate in the high infection group less than either the moderate infection group or the low infection group. All treatment groups had a % cure rate in the moderate infection group less than the low infection group.

The % cure rate for the PZQ-ARA treatment group resulted in a higher % cure rate for the low infection group versus the PZQ-Placebo treatment group or the ARA treatment group (74%, 57% and 50%, respectively). See Table 7.

The % cure rate for the PZQ-ARA treatment group resulted in a higher % cure rate for the moderate infection group versus the PZQ-Placebo treatment group or the ARA treatment group (73%, 70% and 49%, respectively). See Table 8.

The % cure rate for the PZQ-ARA treatment group resulted in a higher % cure rate for the high infection group versus the PZQ-Placebo treatment group or the ARA treatment group (84%, 81 % and 65%, respectively). See Table 9.

Decreased levels of circulating IL-10 and IFN-γ were shown in all treatment groups. Treatment with ARA alone or combined with PZQ was more effective in altering the immune responses of children towards reducing production of II-10 and IFN-γ. These significant decreases in the levels of immunosuppressive IL-10 and IFN-γ indicate a resistance to reinfection with schistosomes. See Tables 10 and 11.

**Table 7: Effect of Treatment with PZQ, ARA or PZQ + ARA**

| **Subjects with initial baseline epg < 100** | | | | |
|---|---|---|---|---|
| **Treatment** | **Number of Children** | **Baseline mean epg** | **% cured** | **% epg reduction*** |
| PZQ | 35 | 38.7 | 57 (20/35) | 50 |
| ARA | 43 | 34.2 | 50 (22/43) | -2.6 |
| PZQ + ARA | 39 | 41.3 | 74 (29/39) | 52 |

| | | | | |
|---|---|---|---|---|
| ***% epg reduction =((mean baseline epg - mean epg 6 weeks after treatment)/mean baseline epg)* 100** | | | | |

**Table 8: Effect of Treatment with PZQ, ARA or PZQ + ARA**

| **Subjects with initial baseline <400 epg > 100** | | | | |
|---|---|---|---|---|
| **Treatment** | **Number of Children** | **Baseline mean epg** | **% cured** | **% epg reduction*** |
| PZQ | 23 | 226.1 | 43 (10/23) | 70 |
| ARA | 29 | 195.6 | 28 (4/19) | 49 |
| PZQ + ARA | 26 | 215.8 | 73 (19/26) | 73 |

| | | | | |
|---|---|---|---|---|
| ***% epg reduction = ((mean baseline epg - mean epg 6 weeks after treatment)/mean baseline epg)* 100** | | | | |

**Table 9: Effect of Treatment with PZQ, ARA or PZQ + ARA**

| **Subjects with initial baseline epg > 400 epg** | | | | |
|---|---|---|---|---|
| **Treatment** | **Number of Children** | **Baseline mean epg** | **% cured** | **% epg reduction*** |
| PZQ | 26 | 979.5 | 30 (8/26) | 81 |
| ARA | 19 | 961.3 | 21 (8/29) | 65 |
| PZQ + ARA | 22 | 805.6 | 68 (15/22) | 84 |

| | | | | |
|---|---|---|---|---|
| ***% epg reduction = ((mean baseline epg - mean epg 6 weeks after treatment)/mean baseline epg)* 100** | | | | |

**Table 10: Effect of Treatment on IL-10 levels with PZQ, ARA or PZQ + ARA**

| **Treatment** | **Number of Children** | **Baseline median IL-10 (pg/mL)** | **Median IL-10 after treatment (pg/mL)** | **% reduction in IL-10 levels** |
|---|---|---|---|---|
| PZQ | 46 | 60 | 36 | 42 |
| ARA | 53 | 80 | 40 | 50 |
| PZQ + ARA | 48 | 70 | 40 | 54 |

**Table 11: Effect of Treatment on IFN-γ levels with PZQ, ARA or PZQ + ARA**

| **Treatment** | **Number of Children** | **Baseline median IFN-γ (pg/mL)** | **Median IFN-γ after treatment (pg/mL)** | **% reduction in IFN-γ levels** |
|---|---|---|---|---|
| PZQ | 46 | 64 | 32.5 | 60 |
| ARA | 53 | 80 | 25 | 70 |
| PZQ + ARA | 48 | 70 | 31 | 56 |

## Claims

1. A composition for use for the treatment and/or prevention of schistosomiasis, to confer resistance to schistosomiasis, and/or to prevent re-occurrence of schistosomiasis, comprising at least one polyunsaturated fatty acid (PUFA) and at least one therapeutic compound, wherein the PUFA is arachidonic acid and wherein the therapeutic compound is 2-(Cyclohexylcarbonyl)-1,2,3,6,7,11b-hexahydro-4H-pyrazino[2,1-a]isoquinolin-4-one (praziquantel), wherein the PUFA is in the form of a free fatty acid, salt, fatty acid ester (e.g. methyl or ethyl ester), monoacylglycerol (MAG), diacylglycerol (DAG), triacylglycerol (TAG), and/or phospholipid (PL).

2. The composition for use according to claim 1, wherein the PUFA is administered in one dosage and the therapeutic compound is administered in a second dosage.

3. The composition for use according to claim 1 or claim 2, wherein the PUFA is administered in an amount of 5-15 mg/kg body weight.

4. The composition for use according to any one of claims 1-3, wherein the therapeutic compound is administered in an amount of 20-60 mg/kg body weight/day.

5. The composition for use according to claim 1, wherein the PUFA and the therapeutic compound are administered in the same dosage.

6. The composition for use according to claim 5, wherein the PUFA is administered in an amount of 10-600 mg/kg body weight/day.

7. The composition for use according to claim 5, wherein the therapeutic compound is administered in an amount of 20-60 mg/kg body weight/day.

8. The composition for use according to any preceding claim, wherein the schistosomiasis is *Schistosoma mansoni.*

9. The composition for use according to any preceding claim, wherein the levels of IL-10 and IFN-γ in an individual are decreased after administration of the PUFA and the therapeutic compound.

10. The composition for use according to any preceding claim, wherein 10 mg/kg body weight/day of the polyunsaturated fatty acid (PUFA) and 40 mg/kg body weight/day of the trematodicide is administered.

11. The composition for use according to any one of claims 1-9, wherein 150 mg/kg body weight/day of the polyunsaturated fatty acid (PUFA) and 40 mg/kg body weight/day of the trematodicide is administered.

12. A composition for the treatment and/or prevention of schistosomiasis, comprising at least one polyunsaturated fatty acid (PUFA) and at least one therapeutic compound, wherein the PUFA is arachidonic acid and wherein the therapeutic compound is 2-(Cyclohexylcarbonyl)-1,2,3,6,7,11b-hexahydro-4*H*-pyrazino[2,1-a]isoquinolin-4-one (praziquantel), wherein the PUFA is in the form of a free fatty acid, salt, fatty acid ester (e.g. methyl or ethyl ester), monoacylglycerol (MAG), diacylglycerol (DAG), triacylglycerol (TAG), and/or phospholipid (PL).

13. The composition of claim 12, wherein the PUFA is administered in one dosage and the therapeutic compound is administered in a second dosage.

14. The composition according to claim 12, wherein the PUFA and the therapeutic compound are administered in the same dosage.

## Patentansprüche

1. Zusammensetzung zur Verwendung bei der Behandlung und/oder Prävention von Schistosomiasis, zum Verleihen von Resistenz gegenüber Schistosomiasis und/oder zum Verhindern eines Wiederauftretens von Schistosomiasis, umfassend mindestens eine mehrfach ungesättigte Fettsäure (Polyunsaturated Fatty Acid, PUFA) und mindestens ein Therapeutikum, wobei es sich bei der PUFA um Arachidonsäure handelt und wobei es sich bei dem Therapeutikum um 2-(Cyclohexylcarbonyl)-1,2,3,6,7,11b-hexahydro-4*H-*pyrazino[2,1-*a*]isochinolin-4-on (Praziquantel) handelt, wobei die PUFA in Form einer freien Fettsäure, eines Salzes, eines Fettsäureesters (z. B. Methyl- oder Ethylesters), als Monoacylglycerin (MAG), Diacylglycerin (DAG), Triacylglycerin (TAG) und/oder Phospholipid (PL) vorliegt.

2. Zusammensetzung zur Verwendung nach Anspruch 1, wobei die PUFA in einer Dosierung verabreicht wird und das Therapeutikum in einer zweiten Dosierung verabreicht wird.

3. Zusammensetzung zur Verwendung nach Anspruch 1 oder Anspruch 2, wobei die PUFA in einer Menge von 5-15 mg/kg Körpergewicht verabreicht wird.

4. Zusammensetzung zur Verwendung nach einem der Ansprüche 1-3, wobei das Therapeutikum in einer Menge von 20-60 mg/kg Körpergewicht/Tag verabreicht wird.

5. Zusammensetzung zur Verwendung nach Anspruch 1, wobei die PUFA und das Therapeutikum in der gleichen Dosierung verabreicht werden.

6. Zusammensetzung zur Verwendung nach Anspruch 5, wobei die PUFA in einer Menge von 10-600 mg/kg Körpergewicht/Tag verabreicht wird.

7. Zusammensetzung zur Verwendung nach Anspruch 5, wobei das Therapeutikum in einer Menge von 20-60 mg/kg Körpergewicht/Tag verabreicht wird.

8. Zusammensetzung zur Verwendung nach einem der vorhergehenden Ansprüche, wobei es sich bei Schistosomiasis um *Schistosoma mansoni* handelt.

9. Zusammensetzung zur Verwendung nach einem der vorhergehenden Ansprüche, wobei die Konzentrationen an IL-10 und IFN-γ in einem Individuum nach Verabreichung der PUFA und des Therapeutikums vermindert sind.

10. Zusammensetzung zur Verwendung nach einem der vorhergehenden Ansprüche, wobei 10 mg/kg Körpergewicht/Tag an mehrfach ungesättigter Fettsäure (Polyunsaturated Fatty Acid, PUFA) und 40 mg/kg Körpergewicht/Tag an Trematodizid verabreicht.

11. Zusammensetzung zur Verwendung nach einem der Ansprüche 1-9, wobei man 150 mg/kg Körpergewicht/Tag an mehrfach ungesättigter Fettsäure (Polyunsaturated Fatty Acid, PUFA) und 40 mg/kg Körpergewicht/Tag an Trematodizid verabreicht.

12. Zusammensetzung zur Behandlung und/oder Prävention von Schistosomiasis, umfassend mindestens eine mehrfach ungesättigte Fettsäure (Polyunsaturated Fatty Acid, PUFA) und mindestens ein Therapeutikum, wobei es sich bei der PUFA um Arachidonsäure handelt und wobei es sich bei dem Therapeutikum um 2-(Cyclohexylcarbonyl)-1,2,3,6,7,11b-hexahydro-4*H-*pyrazino[2,1-*a*]isochinolin-4-on (Praziquantel) handelt, wobei die PUFA in Form einer freien Fettsäure, eines Salzes, eines Fettsäureesters (z. B. Methyl- oder Ethylesters), als Monoacylglycerin (MAG), Diacylglycerin (DAG), Triacylglycerin (TAG) und/oder Phospholipid (PL) vorliegt.

13. Zusammensetzung nach Anspruch 12, wobei die PUFA in einer Dosierung verabreicht wird und das Therapeutikum in einer zweiten Dosierung verabreicht wird.

14. Zusammensetzung nach Anspruch 12, wobei die PUFA und das Therapeutikum in der gleichen Dosierung verabreicht werden.

## Revendications

1. Composition pour une utilisation pour le traitement et/ou la prévention de la schistosomiase, pour conférer une résistance à la schistosomiase, et/ou pour empêcher la réapparition de la schistosomiase, comprenant au moins un acide gras polyinsaturé (PUFA) et au moins un composé thérapeutique, le PUFA étant l'acide arachidonique et le composé thérapeutique étant la 2-(Cyclohexylcarbonyl)-1,2,3,6,7,11b-hexahydro-4*H-*pyrazino[2,1-alpha]isoquinoléin-4-one (praziquantel), le PUFA étant sous la forme d'un acide gras libre, d'un sel, d'un ester d'acide gras (par ex. un ester de méthyle ou d'éthyle), d'un monoacylglycérol (MAG), d'un diacylglycérol (DAG), d'un triacylglycérol (TAG), et/ou d'un phospholipide (PL).

2. Composition pour une utilisation selon la revendication 1, le PUFA étant administré dans un dosage et le composé thérapeutique étant administré dans un deuxième dosage.

3. Composition pour une utilisation selon la revendication 1 ou la revendication 2, le PUFA étant administré en une quantité de 5 à 15 mg/kg de poids corporel.

4. Composition pour une utilisation selon l'une quelconque des revendications 1 à 3, le composé thérapeutique étant administré en une quantité de 20 à 60 mg/kg de poids corporel/jour.

5. Composition pour une utilisation selon la revendication 1, le PUFA et le composé thérapeutique étant administrés dans le même dosage.

6. Composition pour une utilisation selon la revendication 5, le PUFA étant administré en une quantité de 10 à 600 mg/kg de poids corporel/jour.

7. Composition pour une utilisation selon la revendication 5, le composé thérapeutique étant administré en une quantité de 20 à 60 mg/kg de poids corporel/jour.

8. Composition pour une utilisation selon une quelconque revendication précédente, la schistosomiase étant *Schistosoma mansoni.*

9. Composition pour une utilisation selon une quelconque revendication précédente, les taux d'IL-10 et d'IFN-γ chez un individu étant diminués après administration du PUFA et du composé thérapeutique.

10. Composition pour une utilisation selon une quelconque revendication précédente, 10 mg/kg de poids corporel/jour de l'acide gras polyinsaturé (PUFA) et 40 mg/kg de poids corporel/jour du trématodicide étant administrés.

11. Composition pour une utilisation selon l'une quelconque des revendications 1 à 9, 150 mg/kg de poids corporel/jour de l'acide gras polyinsaturé (PUFA) et 40 mg/kg de poids corporel/jour du trématodicide étant administrés.

12. Composition pour le traitement et/ou la prévention de la schistosomiase, comprenant au moins un acide gras polyinsaturé (PUFA) et au moins un composé thérapeutique, le PUFA étant l'acide arachidonique et le composé thérapeutique étant la 2-(Cyclohexylcarbonyl)-1,2,3,6,7,11b-hexahydro-4*H-*pyrazino[2,1-alpha]isoquinoléin-4-one (praziquantel), le PUFA étant sous la forme d'un acide gras libre, d'un sel, d'un ester d'acide gras (par ex. un ester de méthyle ou d'éthyle), d'un monoacylglycérol (MAG), d'un diacylglycérol (DAG), d'un triacylglycérol (TAG), et/ou d'un phospholipide (PL).

13. Composition selon la revendication 12, le PUFA étant administré dans un dosage et le composé thérapeutique étant administré dans un deuxième dosage.

14. Composition selon la revendication 12, le PUFA et le composé thérapeutique étant administrés dans le même dosage.
